# EUROPÄISCHE PATENTSCHRIFT

(11) **EP 3 107 459 B1**
(45) Veröffentlichungstag und Bekanntmachung des Hinweises auf die Patenterteilung: **11.12.2019**
(21) Anmeldenummer: 15707540.9
(22) Anmeldetag: 19.02.2015
(51) Int. Cl.: A61B 8/00, A61B 8/14, A61B 8/15, A61B 8/08

(54) **VORRICHTUNG FÜR DIE ULTRASCHALLGESTÜTZTE REFLEXIONS- UND TRANSMISSIONS- TOMOGRAPHIE**
DEVICE FOR ULTRASOUND-SUPPORTED REFLECTION AND TRANSMISSION TOMOGRAPHY
DISPOSITIF DE TOMOGRAPHIE PAR RÉFLEXION ET TRANSMISSION D'ULTRASONS

(30) Priorität: 20.02.2014 DE 102014102157
(43) Veröffentlichungstag der Anmeldung: 28.12.2016
(73) Patentinhaber: Karlsruher Institut für Technologie (KIT), 76131 Karlsruhe (DE)
(72) Erfinder: GEMMEKE, Hartmut, 76297 Stutensee (DE); ZAPF, Michael, 76149 Karlsruhe (DE); HOPP, Torsten, 67346 Speyer (DE); DAPP, Robin, 75053 Gondeslheim (DE); RUITER, Nicole, 76448 Durmersheim (DE)
(86) Internationale Anmeldenummer: PCT/EP2015/000369
(87) Internationale Veröffentlichungsnummer: WO 2015/124301

(56) Entgegenhaltungen:
- EP-A1- 2 056 124
- US-A1- 2009 230 823
- N.V. RUITER ET AL: "First results of a clinical study with 3D ultrasound computer tomography", 2013 IEEE INTERNATIONAL ULTRASONICS SYMPOSIUM (IUS), 2. Juli 2013 (2013-07-02), Seiten 651-654, XP055191862, DOI: 10.1109/ULTSYM.2013.0168 ISBN: 978-1-46-735684-8
- DIARRA BAKARY ET AL: "Design of Optimal 2-D Nongrid Sparse Arrays for Medical Ultrasound", IEEE TRANSACTIONS ON BIOMEDICAL ENGINEERING, IEEE SERVICE CENTER, PISCATAWAY, NJ, USA, Bd. 60, Nr. 11, 2. November 2013 (2013-11-02), Seiten 3093-3102, XP011529522, ISSN: 0018-9294, DOI: 10.1109/TBME.2013.2267742 [gefunden am 2013-10-16]

## Beschreibung

Die Erfindung betrifft eine Vorrichtung für die Reflexions- und Transmissions-Tomographie mit Ultraschall für zu untersuchende Objekte, insbesondere für Gewebeuntersuchungen von Extremitäten insbesondere der weiblichen Brust gemäß dem ersten Patentanspruch.

Ultraschallgestützte Tomographiesysteme der eingangs genannten Art weisen eine Anordnung von Ultraschallwandlern auf, die um die das zu untersuchende Objekt angeordnet sind und deren Hauptabstrahlungs-richtung auf das Objekt hin gerichtet ist.

DE 28 27 423 A1 beschreibt eine Vorrichtung zur Ermittlung der inneren Struktur eines Körpers mit Hilfe von Schallstrahlen beschreiben, bei dem der Körper in einen mit einem Ankopplungsmedium gefüllten Behälter eingebracht und in diesem mit dem Ultraschalltransmissionsverfahren durchschallt wird. Dabei sind die Ultraschallwandler in einer Matrix zylinderförmig im Behälter angeordnet.

Ebenso offenbaren US 5.673.697 und US 4.478.083 offenbaren Ultra-schallprüfsysteme für die dreidimensionale tomographische Abbildung von Gegenständen, bei denen die Ultraschallwandler matrixförmig um ein Messvolumen angeordnet sind.

In der DE 100 50 232 A1 wird beispielhaft ein hochauflösender Ultra-schalltomograph insbesondere medizinische Untersuchungen beschrieben, bei dem ein zu untersuchendes Körperteil, insbesondere eine weibliche Brust von oben in ein Ultraschallankopplungsmedium in einen offenen Behälter einragt. Die Behälterwandung weist über die gesamte Wandungsfläche ins Behälterinnere orientierte fest angeordnete Ultraschallwandler auf. Diese werden mittels einer rechnergestützten Steuer-, und Auswerteeinheit einzeln oder gruppenweise als Sender oder Empfänger geschaltet, wobei die von den Sendern ausgesendeten Ultraschallsignale Ultraschallimpulse sind. Diese werden auf das zu untersuchende Körperteil geleitet, dort manipuliert und als Reflexions- und Transmissionssignale von allen Empfängern parallel empfangen.

In EP2056124 wird ein Ultraschalltomograph vorgestellt mit einer zufälligen Platzierung von Sendern/Empfängern.

Allgemein wird bei der Gestaltung von ultraschallgestützten Reflexions- und Transmissions-Tomographie angestrebt, durch eine mög-lichst dichte Verteilung von zylindrischen Ultraschallmessköpfen über die Oberfläche einer Halbkugel oder allgemeiner eines rotationssymmetrischen Halbellipsoids um das zu untersuchende Objekt eine möglichst hohe Auflösung zu erreichen. Dabei ist man aufgrund von der möglichst dichten Anordnung der möglichst gleichartigen Ultraschallwandler grundsätzlich bestrebt, diese in einem Muster oder einer periodischen Anordnung zueinander zu positionieren.

Davon ausgehend liegt die **Aufgabe der Erfindung** darin, eine Vorrichtung zur ultraschallgestützten Reflexions- und Transmissions-Tomographie mit erhöhter Bildqualität, insbesondere mit einem verbesserten Bildkontrast und einer Verminderung der Bilodartefakte zu gestalten. Insbesondere soll die Vorrichtung dabei den Erfordernissen einer wirtschaftlichen Fertigung entgegenkommen.

Die Aufgabe wird mit einer Vorrichtung mit den Merkmalen des ersten Patentanspruchs gelöst. Unteransprüche hierzu geben vorteilhafte Ausgestaltungen wieder.

Die Lösung der Aufgabe basiert auf einer Vorrichtung für die ultra-schallgestützte Reflexions- und Transmissions-Tomographie, umfassend ein mit einem Ultraschall-Ankopplungsmediüm gefülltes Messvolumen mit einer Öffnung zum Einsetzen eines zu untersuchenden Körpers wie beispielsweise ein Körperteil wie z.B. eine weibliche Brust oder eine andere Extremität des menschlichen Körpers. Das Messvolumen ist in einem Behälter angeordnet.

Um das Messvolumen ist eine Gesamtzahl von Ultraschallwandlern angeordnet, die in unmittelbarem Kontakt zum Ultraschall-Ankopplungsmedium stehen, um das Messvolumen abseits der Öffnung angeordnet sind und in das Messvolumen ausgerichtet sind. Ein Ultraschallwandler umfasst vorzugsweise ein Schwingelement und ein Ankopplungsmittel zur Umgebung, vorzugsweise einen piezoelektrischen Schwingkörper mit einer Auskopplungsschicht in Schallabstrahlungsrichtung.

Das Messvolumen ist abseits der vorzugsweise oben liegenden Öffnung durch eine Mantelfläche begrenzt, die entweder durch eine nicht materielle imaginäre Ebene oder vorzugsweise durch eine Wandung, vorzugsweise einer Behälterwandung gebildet wird. Vorzugsweise auf dieser Mantelfläche sind die Ultraschallwandler angeordnet, weiter bevorzugt in der Weise, dass die Auskopplungsschichten der Ultraschallwandler auf der Mantelflächenebene liegen oder diese tangieren. Die Ultraschallwandler sind in das Messvolumen hinein ausgerichtet. Die Mantelfläche ist vorzugsweise rotationssymmetrisch oder sphärisch gestaltet und wird weiter bevorzugt durch eine Halbkugel oder Halbellipsoid gebildet.

Zum Betrieb der Vorrichtung werden die Ultraschallwandler vorzugs-weise wie eingangs beschrieben mit einer rechnergestützten Steuer- und Auswerteeinheit-vorzugsweise einzeln als Sender oder Empfänger geschaltet. Die dabei von den Sendern ausgesendeten Ultraschallsignale sind Ultraschallimpulse. Eine bevorzugte betriebliche Ausgestaltung sieht vor, die Wandler sowohl als Sender und Empfänger zu nutzen, wobei die Sender nach Aussenden eines Ultraschallsignals zu einem Empfänger umgeschaltet werden, d.h. für den Empfang des ausgesendeten Ultraschallsignals ertüchtigt werden. Die Vorrichtung ist somit eine wesentliche Komponente eines Ultraschalltomographen.

Ein wesentliches Merkmal ist die Anordnung der Ultraschallwandler um das Messvolumen, die aperiodisch einer zufälligen Gleichverteilung folgt. Dies bedeutet auch, dass jede Position um das Messvolumen in der beschriebenen Anordnung mit einer gleichen Wahrscheinlichkeit von einem Ultraschallwandler besetzt ist. Dazu wird eine Gesamtzahl von vorzugsweise von mindestens 1000, weiter bevorzugt mindestens 2000 und höchstens 10000, weiter bevorzugt 5000, 3000 oder 2500 Ultraschallwandler vorgesehen. Durch die zufällig gleichverteilte Anordnung weisen die Ultraschallwandler Abstände zu direkt benachbart angeordneten Ultraschallwandler auf, die keine ausgeprägten Maxima aufweisen, sondern ebenfalls gleichverteilt in einem Werteintervall für die Abstände befinden. Die genannten Abstände liegen im Beispiel einer Vorrichtung für die Mammographie mit Ultraschall bevorzugt in den Wertebereichen zwischen 1 und 30 mm, bevorzugt zwischen 2 und 10 mm sowie weiter bevorzugt zwischen 3 und 8 mm. Um die Zufälligkeit der Verteilung zu gewährleisten, sollen die Abstände der Ultraschallwandler über einen großen Bereich streuen.

Durch die aperiodisch zufällige Gleichverteilung der Abstände zwischen jeweils unmittelbar benachbarten Ultraschallwandlern verringert man die Wahrscheinlichkeit von Abstrahlungsmaxima und -minima der Ultraschallsignale. Bei der Rekonstruktion häufen sich damit die Ortskurven (Ellipsen) nur noch statistisch. Die Erfindung erhöht damit den Kontrast zumindest um einen Faktor 6 gegenüber der in DE 100 50 232 A1 beschriebenen Anordnung. Die Ultraschallwandler weisen bei einer aperiodisch zufällige Gleichverteilung aber keine periodische Anordnung oder gleiche Abstände zueinander auf. Eine periodische Auslöschung oder Addition von Signalen durch Überlagerung erfolgt folglich nicht mehr unter bestimmten Abstrahlungswinkeln zu allen Wandlern statt, sondern erfolgen für jeden Sendewandlerabstand individuell. Periodisch bedingte Störgrößen wie diese Strahlungsmaxima und -minima (Grating Lobes) werden durch eine ebenso aperiodisch zufällige Gleichverteilung der Wandleranordnung verteilt, was die Bandbreite dieser Störgrößen verringert und eine grundsätzlich verbesserte Trennung von Nutzsignalen und Störgrößen und damit eine verbesserte Bildqualität und einen besseren Kontrast ermöglicht. Insbesondere addieren sich diese Störgrößen nicht mehr zu diskreten Maxima und Minima an bestimmten Winkeln, sondern sind über den gesamten Winkelbereich verteilt und nähern sich in ihrer Summe der Häufigkeit ihres Auftretens einem kontinuierlich verlaufenden Mittelwert an.

Alle Ultraschallwandler sind in das Messvolumen hinein ausgerichtet, d.h. deren Auskopplungsschichten weisen zum Messvolumen hin und stehen in direktem Kontakt mit der Ultraschall-Ankopplungsmedium, vorzugsweise einer Flüssigkeit, vorzugsweise einer wässrigen Lösung oder einem Ultraschall-Ankopplungsgel.

Im Rahmen einer ersten Ausgestaltung sind die Ultraschallwandler mit ihrer jeweiligen Hauptabstrahlungsrichtung orthogonal in das Messvolumen hinein ausgerichtet.

Eine alternative Ausgestaltung sieht vor, dass die Häuptabstrahlungsrichtungen nicht orthogonal, d.h. bei einer sphärischen. Mantel-fläche zum Sphärenmittelpunkt hin auszurichten, sondern in verschiedene Richtungen vorzugsweise zufällig gleichverteilt ausrichten. Damit wird die in das Messvolumen eingebrachte Ultraschallenergie sich nicht auf einen Punkt, nämlich den Sphärenmittelpunkt fokus-siert, sondern gleichmäßig in das Messvolumen oder einen zentralen Teil desselben (z.B. auf das Volumen des zu untersuchenden Körpers im Messvolumen) verteilt.

Die um das Messvolumen vorzugsweise auf der Mantelfläche aperiodisch zufällig gleichverteilt angeordneten Ultraschallwandler sind auf eine Vielzahl von Wandler-felder aufgeteilt, wobei die Anordnungen der Ultraschallwandler auf jedem Wandlerfeld einer aperiodisch einer zufälligen Gleichverteilung folgt. Vorteilhaft dabei ist auch, dass die Ultraschallwandler kein rotationssymmetrisches Anordnungsmuster aufspannen. Jedes Wandlerfeld enthält eine Gruppe von Ultraschallwandlern. Vorzugsweise weisen die Ultraschallwandler eines Wandlerfeldes eine gemeinsame Auskopplungsschicht auf, die sich über die Schwingelemente aller Ultraschallwandler des Wandlerfeldes erstreckt.

Die Ultraschallwandlern und damit die Wandlerfelder sind um das Messvolumen vorzugsweise auf der Mantelfläche angeordnet. Dabei sind die Wandlerfelder nebeneinander angeordnet, d.h. sie überdecken sich nicht gegenseitig. Es wird eine möglichst hohe Oberflächenbedeckung, d.h. dichte Anordnung an Wandlerfeldern auf der bevorzugt sphärischen Mantelfläche angestrebt, weswegen sich jeweils benachbarte Wandler-felder soweit wie geometrisch möglich nahe bis hin zu einer Berührung nebeneinander angeordnet werden.

Grundsätzlich sind die Wandlerfelder kreisförmig.

Eine möglichst hohe Oberflächenbedeckung erzielt man mit Wandlerfeldern unterschiedlicher Form und Größe, beispielsweise mit einer wie vorgenannt möglichst eng geometrisch nah aneinander angeordneten ersten Wandlerfeldfraktion, wobei in den bei der Anordnung entstehenden Zwischenräumen geometrisch kleiner angeordneten zweiten und ggf. weiteren Wandlerfeldfraktion eingesetzt ist. Beispielsweise weist eine mögliche Ausführung, die nicht beansprucht wird, eine nach dem Vorbild eines Fußballs eine erste Wandlerfraktion eine Pentagonform und eine zweite Wandlerfraktion eine Hexagonform auf.

Die Wandlerfelder sind in ihrer Form gleich, vorzugsweise auch in deren Grösse.

Besonders vorteilhaft für eine wirtschaftliche Fertigung ist eine auf jedem Wandlerfeld identische Anordnung der Ultraschall-wandler. Die Wandlerfelder und damit Sensoren weisen damit alle die gleiche (aperiodisch zufällige gleichverteilte) Anordnung mit nicht rotationssymmetrischen Anordnungsmustern der Ultraschallwandler auf und sind damit einfacher in Serie herstellbar. Um dabei eine Periodizität bei einer Aneinanderreihung von identischen Sensoren zu vermeiden, werden diese und damit die Wandlerfelder mit den Anordnungsmustern der Ultraschallwandler gegeneinander verdreht eingesetzt, vorzugsweise mit einer individuellen zufälligen Drehung zueinander.

Gemäss der vorliegenden Erfindung, sind die Wandlerfelder kreisförmig gestaltet, die den Vorteil der stufenlosen und damit gleichverteilbaren Verdrehung der Wandlerfelder zueinander zulässt. Durch diese gleichverteilbaren Verdrehbarkeit mit den maximal möglichen Freiheitsgraden einer Verstellbarkeit der Verdrehwinkel der Drehung wird im Gegensatz zu eckigen Wandlerfelder (Eckenanzahl entspricht meist der Anzahl der einstellbaren Verdrehungswinkel) eine zusätzliche Sicherheit gegenüber sich zufällig ergebenden periodischen Anordnungen durch benachbarte Wandlerfelder erzielbar.

Die zufällige Gleichverteilung der Ultraschallwandler auf der Mantel-fläche wird mit dem vorgenannten Konzept auf dem Wandlerfeld lokal und darüber hinaus durch zufällige Rotation und dichteste Packung der Wandlerfelder auf der Mantelfläche realisiert. Mit der vorgenannten Verteilung der Ultraschallwandler lassen sich bei gleichzeitiger Nutzung aller Wandler-Positionen zum Senden und Empfangen, mit einer Position der Messanordnung 5·10⁶ Messspektren (A-Scans) aufnehmen. Das ist hinreichend um ein vollständiges 3D-Bild in der Vorrichtung erstellen. Herkömmliche Systeme benötigten hierzu zumindest sechs Rotations- und Hub-Positionen der Messanordnung um hinreichende Bildqualität zu erreichen. Im Vergleich zu diesen lässt sich ein um mindestens einen Faktor 6 höheren Kontrast in der Reflexionstomographie und Faktor 2 bessere Bildqualität in der Transmissions-tomographie erzielen. Damit einher gehen kürzere Datenaufnahmezeit im Bereich von wenigen Minuten, in denen sich auch die mechanischen Bewegungen der Messanordnung sich auf 1 - 3 reduzieren lassen.

Die Erfindung und weitere Details und Ausgestaltungen werden im Folgenden anhand von Ausführungsbeispielen und mit Figuren näher erläutert. Es zeigen
**Fig.1a** **bis d** schematische Darstellungen von Ausführungen der Vorrichtung,
**Fig.2a** **und b** jeweils eine dreidimensionale Darstellung von Ultraschallwandlern um ein Messvolumen,
**Fig.3** eine dreidimensionale Darstellung von kreisförmigen Wandlerfeldern in einer bevorzugten dichtesten Anordnung um ein Messvolumen,
**Fig.4a** **bis c** jeweils eine graphischen Darstellung der maximal erzielbaren relativen Oberflächenbedeckung **(a)**, der maximalen Sensoranzahl **(b)** und maximalen Winkel θ **(c)** in Abhängigkeit des Durchmessers des Sensors für eine Ausführung gemäß **Fig.1a** mit 175 mm Radius,
**Fig.5** eine graphische Darstellung der Verteilung des Winkels θ in Abhängigkeit des Winkels θ für das Ausführungsbeispiel in **Fig.4****,**
**Fig.6** eine prinzipielle Draufsicht eines beispielhaften Anordnungsmusters von aperiodisch zufällig auf einem runden Wandlerfeld gleichverteilten Ultraschallwandlern,
**Fig.7a** **und b** die Verteilungen der Ultraschallwandler in Abhängigkeit der Winkels θ einer herkömmlichen Anordnung von Ultraschallwandlern **(a,** vgl. **Fig.2a****)** und einer aperiodisch zufällig gleichverteilten Anordnung von Ultraschallwandlern **(b,** vgl. **Fig.2b****)** sowie
**Fig.8a** **und b** je eine Schnittdarstellung durch eine bevorzugten Ausführung eines Wandlerfelds eines Wandlerelements mit Ultra-schallwandern mit gemeinsamer Auskopplungsschicht.

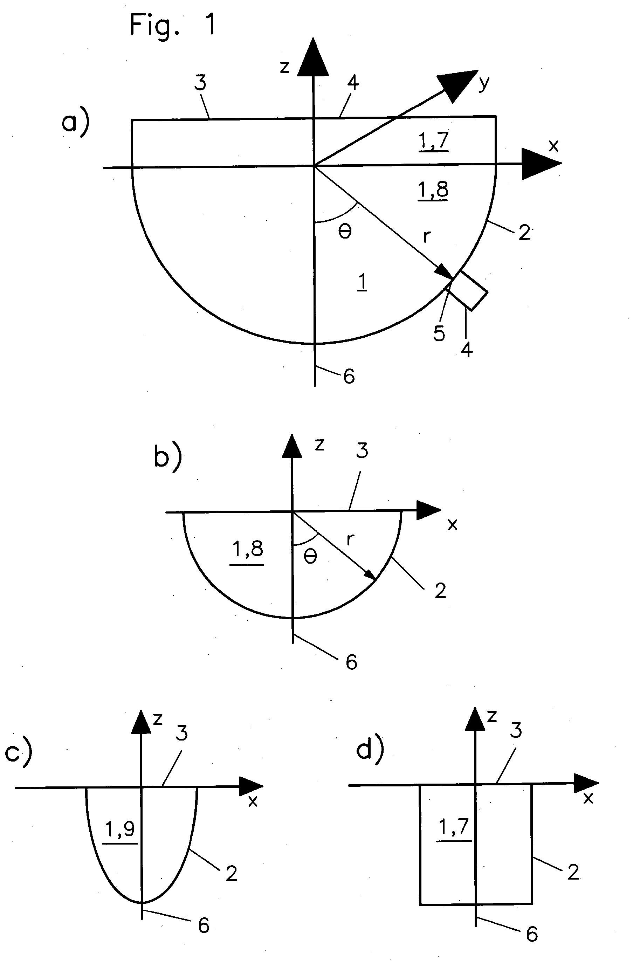

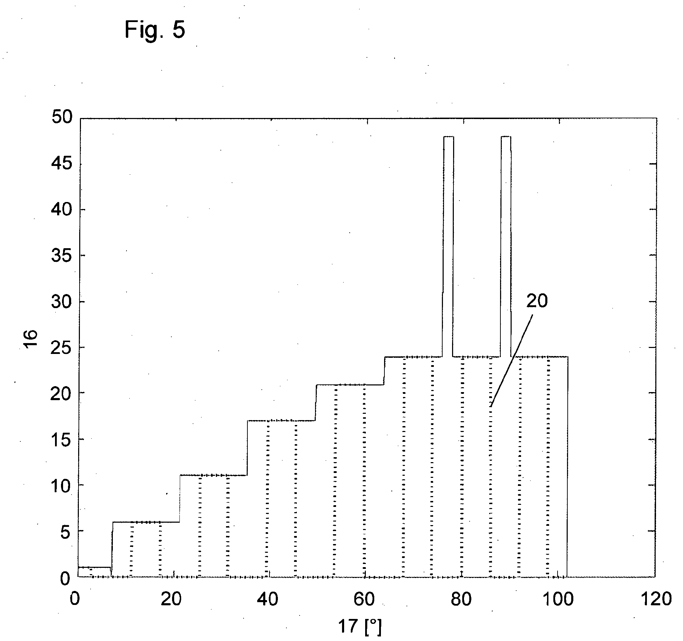

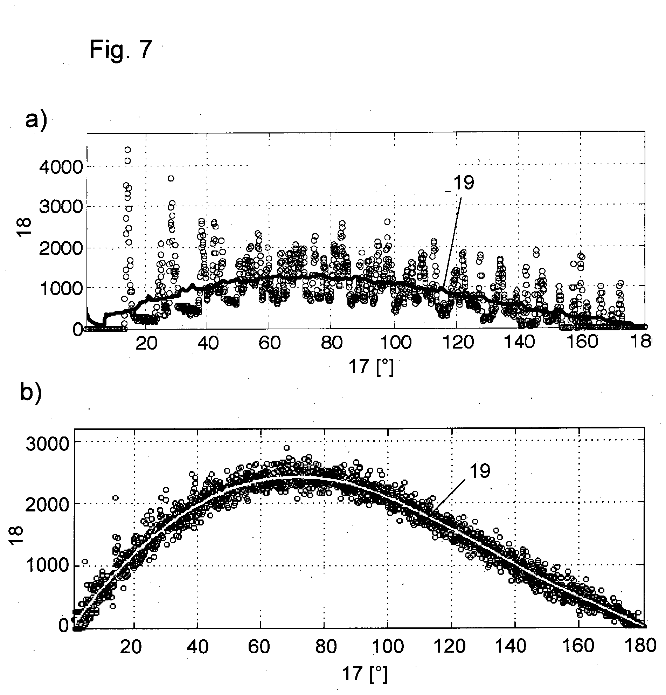

Die in **Fig.1a** **bis d** dargestellten Ausführungen zeigen mögliche Ausgestaltungen des Messvolumens **1** jeweils mit Mantelflächen **2** und oben liegender Öffnung **3,** die vorzugsweise rotationssymmetrisch um eine Symmetrieachse **6** erstrecken. Die Mantelflächen werden bevorzugt als Gefäßwandung und Haltestruktur für die Wandlerelemente **4** gestaltet, wobei deren Wandlerflächen **5** mit den darin eingesetzten Ultraschallwandlern vorzugsweise plan auf der Mantelfläche angeordnet sind. Die dargestellten Messvolumina weisen zylindrische Anteile **7** und/oder sphärische Anteile **8** und/oder ellipsoide Anteile **9** auf. Ein zylindrischer Anteil zeichnet sich anders als die sphärischen und ellipsoiden Anteile vorzugsweise durch direkt auf einer Ebene gegeneinander gerichtete Ultraschallwandler aus und eignet sich insbesondere für die Transmissions-Ultraschalltomographie. Dagegen eigenen sich sphärische und ellipsoide Anteile insbesondere für eine Erfassung großer Bereiche des Winkels θ (vgl. **Fig.5****,** **7a und 7b****)** und damit für die Reflexions- und Streuungssignal-Tomographie.

**Fig.1a** zeigt ein Messvolumen **1** mit einem zylindrischen Anteil **7** und einem halbkugelförmigen sphärischen Anteil **8** mit einem Krümmungsradius **r,** ausgehend von der Symmetrielinie **6.** Einen gleichen als Halbkugel gestalteten sphärischen Anteil **8** des Messvolumens **1,** jedoch ohne den zylindrischen Anteil zeigt die Ausführung gemäß **Fig.1b****.**

**Fig.1c** zeigt eine Ausführung mit einem Messvolumen mit ellipsoider Mantelfläche, d.h. ausschließlich mit einem ellipsoiden Anteil des Messvolumens **9.** Durch die schlankere und im unteren Bereich vorzugs-weise rund zusammenlaufenden Form des Messvolumens schränkt sich der für die Reflexions- und Streuungssignal-Tomographie günstige Winkelbereich ein. Anordnungen mit Ultraschallwandlern mit kleinen Winkeln θ sind nur noch in einer geringeren Anzahl vorhanden, während Anordnungen mit den für die Transmissions-Ultraschalltomographie günstigen Winkelbereichen mit Winkeln θ um 90±30°, vorzugsweise 90±10° größere Mantelflächenbereiche einnehmen. Weiterhin ist diese Ausführung auch mit einem nicht eigens dargestellten zylindrischen Anteil im Bereich der Öffnung kombinierbar (ähnlich **Fig.1a**).

Schließlich zeigt **Fig.1d** ein Messvolumen mit ausschließlich zylindrischem Anteil **7** speziell für die Transmissions-Tomographie.

Weitere nicht in Figuren bildlich dargestellte Ausgestaltungen eines um die Symmetrielinie rotationssymmetrischen Messvolumens sehen eine eckige Approximation der vorgenannten Querschnitte des Messvolumens vor, beispielsweise umfassend einen zylindrischen Anteil **7,** an den sich anstelle des in **Fig.1a** dargestellten spärischen Anteils **8** eine oder mehrere Kegelstupfförmige Anteile anschließen und im unteren Bereich spitz zusammenlaufen oder vorzugsweise durch eine ebene Kreisfläche abgeschlossen sind.

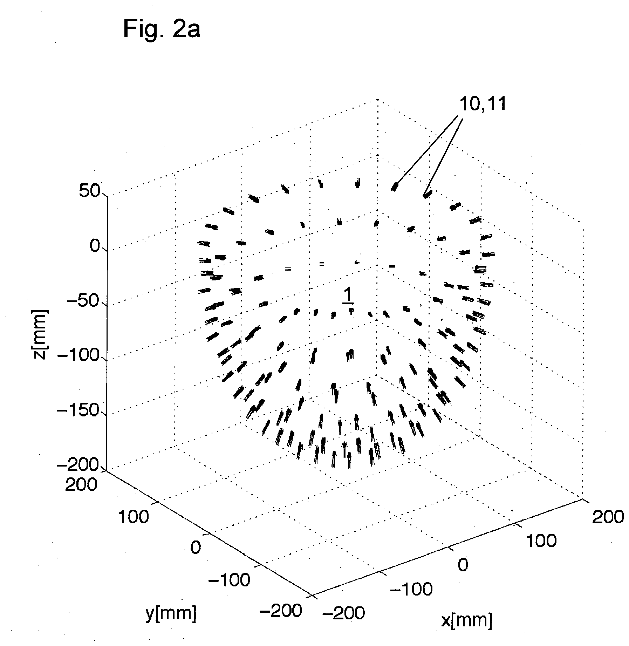

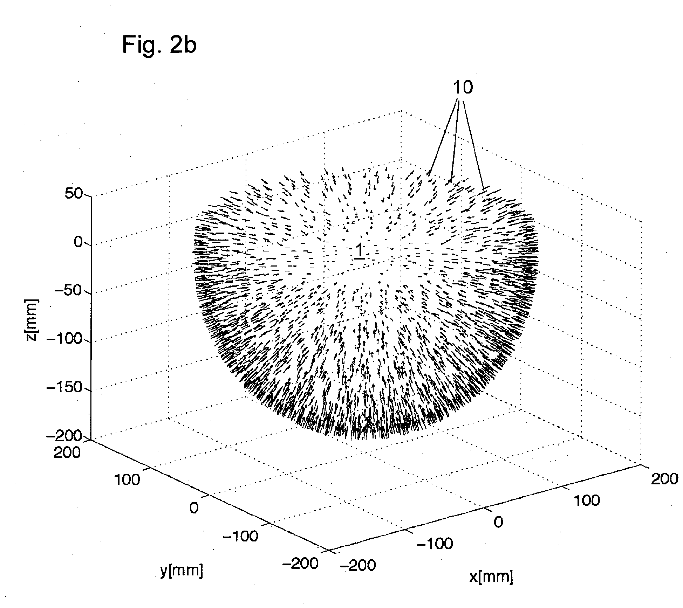

**Fig.2a** **und b** geben beispielhaft je eine perspektivische Darstellung von Ultraschallwandlern **10** um ein Messvolumen **1** in einer in **Fig.1a** wiedergegebenen Geometrie wieder. Der Krümmungsradius r beträgt im Beispiel 175 mm, die Höhe des zylindrischen Anteils des Messvolumens 211,5 mm.

**Fig.2a** repräsentiert mit einer Zusammenfassung der insgesamt 2028 Ultraschallwandler **10** zu Ultraschallwandlergruppen **11** mit quadratischer Anordnung (z.B. 156 Ultraschallwandlergruppen mit je neun Empfangs-wandler und vier Sendewandler in quadratischer Formation mit ca. 5 mm Kantenlänge entsprechend einer schachbrettmusterähnlichen Ineinanderschachtelung), die wiederum in gleichen Abständen zueinander angeordnet sind. Diese periodische Anordnung lokal wie auch gesamtheitlich entspricht dem im vorgenannten Stand der Technik beschriebenen.

**Fig.2b** zeigt dagegen eine Anordnung von ca. 2300 Ultraschallwandlern, die einer aperiodischen zufälligen Gleichverteilung unter Vermeidung periodischer auftretenden Abständen folgt. Die Abstände benachbarter Ultraschallwandler betragen im Beispiel mit gleicher Verteilungswahrscheinlichkeit zwischen 2,4 und 9,2 mm zueinander.

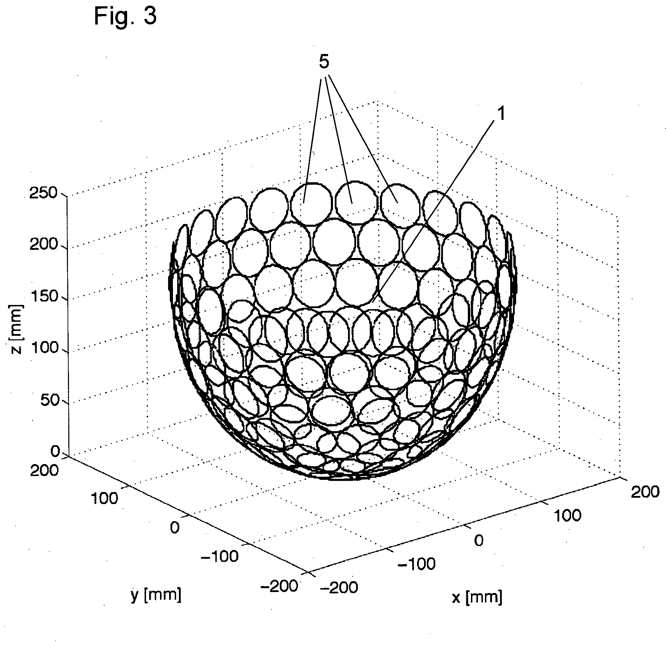

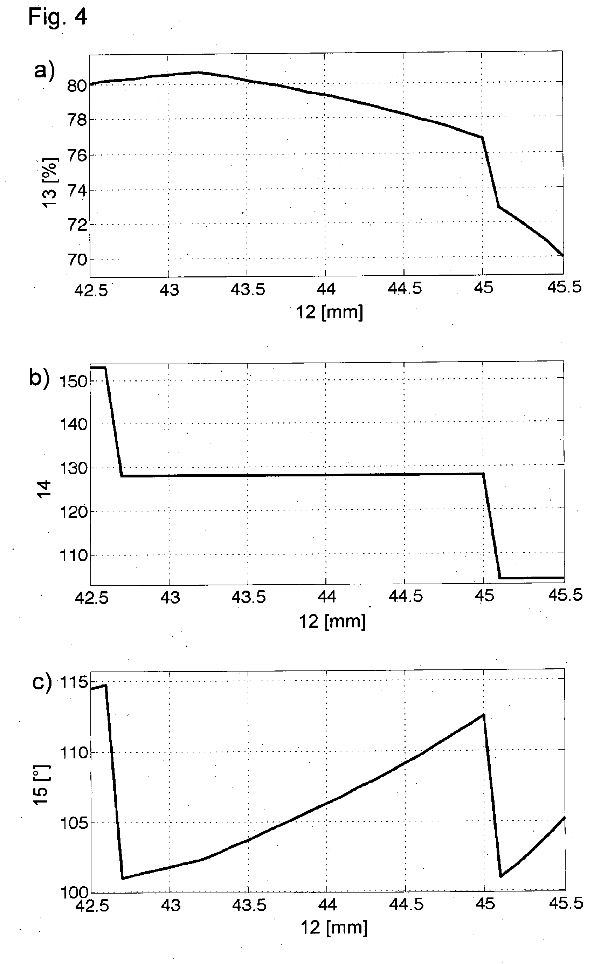

Die gleichverteilten Ultraschallwandler sind wie zuvor beschrieben in Wandlerfelder angeordnet. **Fig.3** zeigt beispielhaft eine möglichst dichte Anordnung von runden Wandlerfeldern **5** auf einer Mantelfläche des in **Fig.2a** **und b** dargestellten Messvolumens **1** (Krümmungsradius **r** = 175 mm). Jedes Wandlerfeld ist rund weist einen vorbestimmten gleichen Durchmesser auf, und jeder Ultraschallwandler befindet in bei dieser Ausführung auf einem der Wandlerfelder. Weiter bevorzugt sind alle Wandlerfelder und insbesondere deren Anordnung und Anzahl der Ultraschallwandler auf diesen identisch, wobei die geometrische Anordnung von Ultraschallwandlern auf den Wandlerfeldern einer aperiodischen zufälligen Gleichverteilung folgt. Eine Periodizität der Wanderfelder wird durch eine zufällig gleichverteilte Verdrehung benachbarter Wandlerfelder vermieden. Wesentlich für eine angestrebte dichtest mögliche Oberflächenabdeckung **13** der in **Fig.1a** **und** **3** dargestellten Mantelfläche durch runde Wandlerfelder ist die Einstellung eines bestimmten Verhältnisses aus Krümmungsradius **r** und Durchmesser der Wandlerfelder **12,** wie er aus **Fig.4a** ermittelbar ist. Bei einem Krümmungsradius **r** von 175 mm ist mit Wandlerfeldern mit 43,2 mm Durchmesser eine maximale Oberflächen-abdeckung von 80,7% erzielbar, was einem Verhältnis von Krümmungsradius **r** zu Durchmesser **12** von 4,05 entspricht. Sehr hohe Oberflächenabdeckungen über 80% werden im Beispiel noch mit Durchmessern der Wanderlfelder zwischen 42,5 mm und 43,6 mm erzielbar, womit sich das vorgenannte Verhältnis zu Werten im bevorzugten Intervall zwischen 4,01 und 4,12 berechnet. Andere Ausgangsgrößen für den Krümmungsradius und den Wandlerfelddurchmesser führen zu gleichen relativen Oberflächenabdeckungen, wenn diese vorgenannten Verhältnisse und damit die Proportionen zueinander gewahrt bleiben. **Fig.4b** zeigt den stufenförmigen Verlauf der auf der Mantelfläche unterzubringenden Wandlerzahl **14** über den Durchmesser der Wandlerfelder **12.** Mit den Stufen ändert sich auch der maximal erzielbare und in **Fig.1a** dargestellte Winkel θ **15** stufenweise, wobei mit der Zunahme der Anzahl der Wanderfelder aus **Fig.4b** auch der maximal erzielbare Winkel **15** steigt **(****Fig.4c****).**

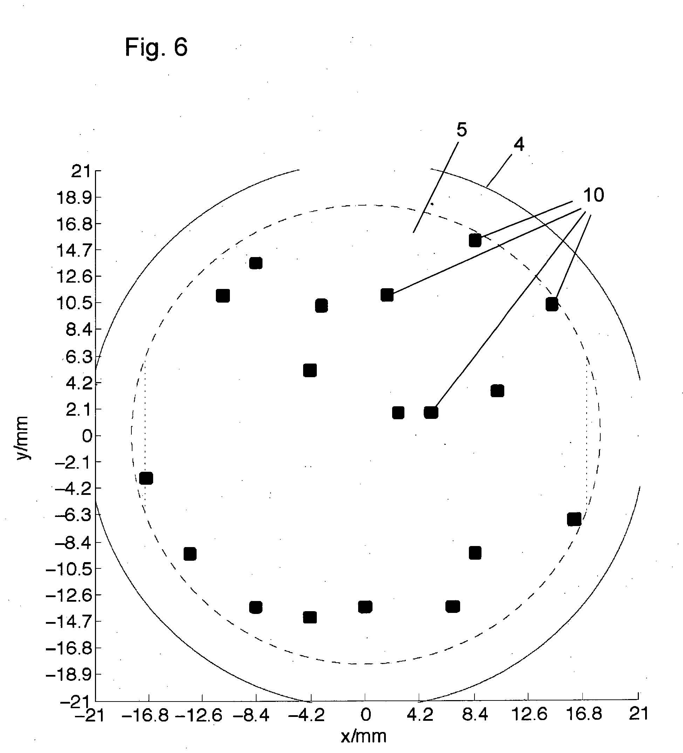

**Fig.5** stellt im Beispiel gemäß **Fig.2b** die Verteilung der Wandler **16** über die Verteilung des Winkels θ **17** in einem Histogramm auf (durch-gezogene Linie), wobei aufgrund des zylindrischen Anteils des Mess-volumens der Bereich über 70° hoch ausfällt. In dem Bereich der Gürtellinie, d.h. dem differenzierbar stetigen Übergang von der Halbkugel zum Zylinder hat die Optimierung drei verschränkt angeordnete Ringe mit jeweils 24 Wandlerfeldern untergebracht (vgl. **Fig.3****).** Durch die Wanddicke und den minmalen Abstand der Sensoren zur Wand der ebenen Wandlerfelder gemäß **Fig.6** schränkt sich der benutzbare Bereich **20** ein, und zwar auf die mit gestrichelter Linie angegebenen Bereiche.

Eine beispielhafte Aufsicht auf ein rundes Wandlerfeld **5** mit 43,2 mm Durchmesser und darin 18 gleichverteilte Ultraschallwandlern **10** zeigt **Fig.6****.** Das vorzugsweise plane Wandlerfeld bildet den Abschluss eines zylindrischen Wandlerelements in einem rohrförmigen Gehäuse, dessen Innenwandung gestrichelt dargestellt ist. Vorzugsweise weisen alle Ultraschallwandler eine gemeinsame Auskopplungsschicht auf, die sich über das gesamte Wandlerfeld erstreckt.

**Fig.7a** **und b** zeigen die Verteilungen der Ultraschallwandler **18** auf die Winkel θ **17** einer herkömmlichen Anordnung von Ultraschallwandlern **(****Fig.7a****,** vgl. **Fig.2a****)** und einer aperiodisch zufällig gleichverteilten Anordnung von Ultraschallwandlern auf Wandlerfeldern **(****Fig.7b****),** wie diese beispielhaft in **Fig.3b** und **Fig.6** wiedergegeben ist. Das Messvolumen und dessen Abmessungen entsprechen dem in **Fig.2a** **und b** sowie **Fig.3** beschriebenen. Ohne Gleichverteilung zeigen sich deutliche Maxima und Minima, die sich zudem periodisch über den gesamten Winkelbereich erstrecken. Die Varianzen der Verteilungswerte um die jeweiligen Medianwertverlauf **19** berechnen sich zu 2,56·10⁵ für die herkömmliche Anordnung gemäß **Fig.2a** und reduziert sich zu 3,98.10³ für die gleichverteilte Anordnung der Ultraschallwandler um das Messvolumen.

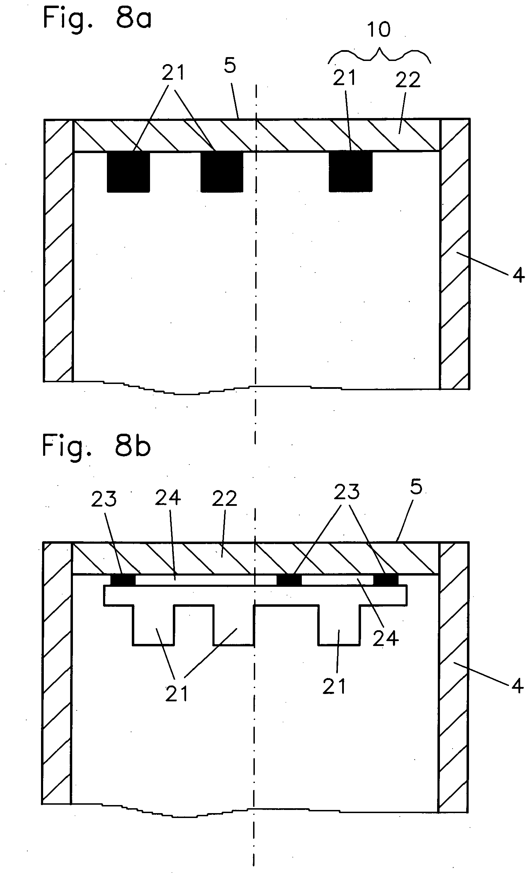

**Fig.8a** **und b** zeigen je eine Schnittdarstellung durch bevorzugte Aus-führungen eines Wandlerfelds **5** eines Wandlerelements **4** mit Ultra-schallwandern mit gemeinsamer Auskopplungsschicht. Ein Ultraschall-wandler umfasst dabei ein piezoekeramisches Schwingelement **21** mit jeweils mindestens einer unteren und einer oberen Elektrode und ein Auskopplungsmittel, das durch eine gemeinsame Auskopplungsschicht **22** für alle Schwingelemente des Wandlerelements gebildet wird und das gesamte Wandlerfeld **5** überspannt. Im beschriebenen Ausführungsbeispiel besteht die Auskopplungsschicht **22** vorzugsweise insgesamt, zumindest aber teilweise aus einer Platine, auf die die Schwingelemente **21** jeweils mit einer ihrer Elektroden verbunden sind. Auf den Platinen sind vorzugsweise Leiterbahnstrukturen mit konstanter Leiterbahnhöhe unter den piezoelektrischen Schwingelementen vorgesehen, die in einem elektrischen Kontakt mit einer Elektrode des Schwingelements stehen.

**Fig.8a** zeigt die Ausgestaltung, bei der die Schwingelemente mit einer Elektrode einzeln auf die Leiterbahnen der Platine aufgesetzt und beispielsweise als SMD-Bauelement mit bekannten Mittel beispielsweise elektrisch leitenden Klebstoff verbunden werden. Die Leiterbahnen dienen dabei als Abstandshalter.

**Fig.8b** zeigt dagegen eine alternative Ausgestaltung, bei der die Schwingelemente als Erhebungen eines monolithischen Formelements aus einem piezoelektrischen Material hervorstehen und über Abstandselemente **23** abseits der Schwingelemente in einem vorgegebenen Abstand zur Platine positioniert werden. Der verbleibende Hohlraum **24** zwischen Platine und piezoelektrischen Schwingelement **21** ist neben der Leiterbahnstruktur vollständig durch ein Bindemittel, beispielsweise einem elektrisch leitenden Klebstoff aufgefüllt.

Die Platine sowie das Bindemittel weisen akustische Impedanz auf, die zwischen der der piezoelektrischen Schwingkörper und dem Ultraschall-Ankopplungsmedium im Messvolumen liegt.

Ein wesentliche Grundgedanke insbesondere der letztgenannten Ausgestaltungen besteht darin, die piezoelektrischen Schwingelemente mit einer der beiden planparallel zueinander angeordneten Elektrodenfläche auf Abstandselementen in einfacher Weise exakt ausgerichtet, elektrisch kontaktierend, planparallel und reproduzierbar auf eine Platine zu positionieren und zu fixieren. Dies erleichtert in vorteilhafter Weise eine Serienfertigung von mit Ultraschallwandler bestückten Wandlerfelder als wesentliche Komponente der Wanderelemente. Auf der Platine befindet sich zu diesem Zweck bevorzugt eine aus einer flächigen Platinenbeschichtung herausgeätzte oder über eine Dickfilmtechnik (z.B. mit Siebdruck) aufgebrachte Leiter-bahnstruktur. Diese dient einerseits als elektrische Kontaktierung mit der Elektrodenfläche, andererseits mit ihrer exakten und konstanten Leiterbahnhöhe als Abstandselemente zwischen Platine und piezoelektrischen Schwingelementen, und zwar über den gesamten Elektrodenbereich erstreckend. Zur Fixierung des piezoelektrischen Körpers auf der Platine eignen sich Lote oder Klebstoffe, die bei einem Zusammenpressen der piezoelektrischen Schwingelemente und Platine lokal in die neben den Leiter-bahnstruktur verbleibenden Hohlräume zwischen Platine und dem Schwingelement ausweichen und diese dabei vollständig ausfüllen. Das Aufbringen der Schwingelemente erfolgt durch ein Anpressen dieses auf die Leiterbahnstruktur, wobei der Klebstoff oder das Lot an mindestens einer Stelle durch das Aufeinandertreffen der Leiterbahnstruktur und der Elektrodenfläche durchkontaktiert wird. Die Leiterbahnstruktur dient damit gleichzeitig als elektrischer Anschluss für die abstrahlungsseitige Elektrodenfläche. Die Dicke des gesamten Auskopplungsschichtsystem, bestehend aus Platine und Bindemittel im Hohlraum beträgt idealer Weise ein Viertel der Ultraschallfrequenz λ/4, auf die der Ultraschallwandler ausgelegt ist.

### Bezugszeichenliste

- **1**: Messvolumen
- **2**: Mantelfläche
- **3**: Öffnung
- **4**: Wandlerelement
- **5**: Wandlerfeld
- **6**: Symmetrielinie
- **7**: zylindrischer Anteil des Messvolumens
- **8**: sphärischer Anteil des Messvolumens
- **9**: ellipsoider Anteil des Messvolumens
- **10**: Ultraschallwandler
- **11**: Ultraschallwandlergruppe
- **12**: Durchmesser des Wandlerfeldes
- **13**: Oberflächenabdeckung
- **14**: Wandlerzahl
- **15**: Maximaler Winkel θ
- **16**: Verteilung der Wandler
- **17**: Winkel θ
- **18**: Verteilungen der Ultraschallwandler
- **19**: Medianwertverlauf
- **20**: Benutzbarer Bereich
- **21**: Schwingelement
- **22**: Auskopplungsschicht
- **23**: Abstandselement
- **24**: Hohlraum, angefüllt mit Bindemittel

## Patentansprüche

1. Vorrichtung für die ultraschallgestützte Reflexions- und Transmissions-Tomographie, umfassend
a) ein mit einem Ultraschall-Ankopplungsmedium gefülltes Messvolumen **(1)** mit einer Öffnung **(3)** zum Einsetzen eines zu untersuchenden Körpers sowie
b) eine Gesamtzahl von Ultraschallwandlern **(11),** die mit unmittelbaren Kontakt zum Ultraschall-Ankopplungsmedium um das Messvolumen abseits der Öffnung angeordnet und in das Messvolumen ausgerichtet sind, wobei das Messvolumen eine Mantelfläche **(2)** abseits der Öffnung aufweist, wobei
c) die Gesamtzahl von Ultraschallwandlern **(11)** auf eine Vielzahl von Wandlerfeldern **(5)** aufgeteilt ist und die Anordnungen der Ultraschallwandler auf jedem Wandlerfeld einer aperiodisch zufälligen Gleichverteilung folgt,
**dadurch gekennzeichnet,dass**
d) die Wandlerfelder (5) kreisförmig sind.

2. Vorrichtung nach Anspruch 1, **dadurch gekennzeichnet, dass** die Gesamtzahl von Ultraschallwandlern **(11)** 2000 übersteigt.

3. Vorrichtung nach Anspruch 1 oder 2, **dadurch gekennzeichnet, dass** die Mantelfläche **(3)** rotationssymmetrisch oder sphärisch gestaltet ist und vorzugsweise durch eine Halbkugel oder Halbellipsoid gebildet wird.

4. Vorrichtung nach einem der vorgenannten Ansprüche, **dadurch gekennzeichnet, dass** die Ultraschallwandler **(11)** auf der Mantelfläche **(2)** angeordnet sind und jeweils eine Hauptabstrahlungsrichtung in das Messvolumen **(1)** hinein aufweisen.

5. Vorrichtung nach einem der vorgenannten Ansprüche, **dadurch gekennzeichnet, dass** die Wandlerfelder **(5)** nebeneinander angeordnet sind und jeweils benachbarte Wandlerfelder berühren.

6. Vorrichtung nach einem der vorgenannten Ansprüche, **dadurch gekennzeichnet, dass** die Wandlerfelder **(5)** geometrisch gleich sind.

7. Vorrichtung nach einem der vorgenannten Ansprüche, **dadurch gekennzeichnet, dass** die geometrische Anordnung und die Anzahl von Ultraschallwandlern **(11)** auf einer überwiegenden Mehrzahl von Wandlerfeldern **(5)** identisch ist, wobei die Wandlerfelder zueinander verdreht angeordnet sind.

8. Vorrichtung nach einem der vorgenannten Ansprüche, **dadurch gekennzeichnet, dass** die Wandlerfelder **(5)** mit einer individuellen zufälligen Drehung zueinander verdreht angeordnet sind.

## Claims

1. Device for ultrasound-supported reflection and transmission tomography, comprising
a) a measurement volume (1) with an opening (3) for inserting a body which is to be examined, and
b) a total number of ultrasonic transducers (11), which are arranged with direct contact to the ultrasonic coupling medium around the measurement volume away from the opening and are oriented into the measurement volume, wherein the measurement volume comprises a jacket surface (2) away from the opening,
wherein
c) the total number of ultrasonic transducers (11) are distributed onto a plurality of transducer fields (5), and the arrangement of the ultrasonic transducers on each transducer field follows an aperiodic random uniform distribution,
**characterised in that**
d) the transducer fields (5) are circular in shape.

2. Device according to claim 1, **characterised in that** the total number of ultrasonic transducers (11) exceeds 2000.

3. Device according to claim 1 or 2, **characterised in that** the jacket surface (3) is configured as rotation symmetrical or spherical, and is preferably formed by a hemisphere or hemi-ellipsoid.

4. Device according to any one of the preceding claims, **characterised in that** the ultrasonic transducers (11) are arranged on the jacket surface (2) and in each case exhibit a main radiation direction into the measurement volume (1).

5. Device according to any one of the preceding claims, **characterised in that** the transducer fields (5) are arranged next to one another, and in each case adjacent transducer fields are in contact.

6. Device according to any one of the preceding claims, **characterised in that** the transducer fields (5) are geometrically the same.

7. Device according to any one of the preceding claims, **characterised in that** the geometric arrangement and the number of ultrasonic transducers (11) are identical on an overwhelming majority of transducer fields (5), wherein the transducer fields are arranged rotated towards one another.

8. Device according to any one of the preceding claims, **characterised in that** the transducer fields (5) are arranged rotated towards one another with an individual random rotation.

## Revendications

1. Dispositif de tomographie par réflexion et transmission assistance ultrasonique comprenant :
a) un volume de mesure (1) rempli d'un fluide de couplage ultrasonique comprenant une ouverture (3) d'introduction d'un corps à analyser, et
b) un nombre global de transducteurs à ultrasons (11) qui sont en contact direct avec le fluide de couplage ultrasonique autour du volume de mesure, à l'écart de l'ouverture, et sont orientés dans le volume de mesure, le volume de mesure comportant une surface-enveloppe (2) située à l'écart de l'ouverture,
c) le nombre global de transducteurs à ultrasons (11) est subdivisé en un ensemble de champs de transducteur (5), et les agencements des transducteurs à ultrasons suit sur chaque champ de transducteur suivent une répartition uniforme aléatoire apériodique,
**caractérisé en ce que**
d) les champs de transducteur (5) sont circulaires.

2. Dispositif conforme à la revendication 1,
**caractérisé en ce que**
le nombre global de transducteurs à ultrasons (11) dépasse 2000.

3. Dispositif conforme à la revendication 1 ou 2,
**caractérisé en ce que**
la surface enveloppe (3) présente une symétrie de rotation ou est de forme sphérique, et est de préférence formée par une demi-sphère ou par un demi-ellipsoïde.

4. Dispositif conforme à l'une des revendications précédentes,
**caractérisé en ce que**
les transducteurs à ultrasons (11) sont installés sur la surface enveloppe (2) et ont chacun une direction de rayonnement principale dans le volume de mesure (1).

5. Dispositif conforme à l'une des revendications précédentes,
**caractérisé en ce que**
les champs de transducteur (5) sont situés côte à côte et des champs de transducteur voisins respectifs sont en contact.

6. Dispositif conforme à l'une des revendications précédentes,
**caractérisé en ce que**
les champs de transducteur (5) ont une géométrie similaire.

7. Dispositif conforme à l'une des revendications précédentes,
**caractérisé en ce que**
l'agencement géométrique et le nombre de transducteurs à ultrasons (11) sont identiques sur un ensemble principal de champs de transducteur (5), les champs de transducteur étant tournés les uns par rapport aux autres.

8. Dispositif conforme à l'une des revendications précédentes,
**caractérisé en ce que**
les champs de transducteur (5) sont tournés les uns par rapport aux autres selon une rotation individuelle aléatoire.
